# EUROPEAN PATENT APPLICATION

(11) **EP 3 636 134 A1**
(43) Date of publication of application: **15.04.2020**
(21) Application number: 18806742.5
(22) Date of filing: 18.05.2018
(51) Int. Cl.: A61B 1/045, A61B 1/00, G06T 1/00, H04N 7/18

(54) **INFORMATION PROCESSING DEVICE, CONTROL METHOD, AND PROGRAM**

(30) Priority: 25.05.2017 JP 2017103348
(71) Applicant: NEC Corporation, Tokyo 108-8001 (JP); National Cancer Center, Tokyo 104-0045 (JP)
(72) Inventor: TAKAHASHI Ikuma, Tokyo 108-8001 (JP); SANO Maki, Tokyo 108-8001 (JP); OKUTSU Motoyasu, Tokyo 108-8001 (JP); TANAKA Chiemi, Tokyo 108-8001 (JP); SAIKOU Masahiro, Tokyo 108-8001 (JP); IMAOKA Hitoshi, Tokyo 108-8001 (JP); KAMIJO Kenichi, Tokyo 108-8001 (JP); SAITO Yutaka, Tokyo 104-0045 (JP); YAMADA Masayoshi, Tokyo 104-0045 (JP)
(74) Representative: Betten & Resch
(86) International application number: PCT/JP2018/019309
(87) International publication number: WO 2018/216617

(57) **Abstract**

An information processing apparatus (2000) detects an abnormal region (30) from video data (12). The information processing apparatus (2000) displays a video frame (14) in which the abnormal region (30) is detected in a first region (22) of a display device (20). Further, the information processing apparatus (2000) displays the video data (12) including the video frame (14) generated after the video frame (14) displayed in the first region in a second region (24) of the display device (20).

## Description

### TECHNICAL FIELD

The present invention relates to an information processing apparatus, a control method, and a program.

### BACKGROUND ART

An examination is performed to find out whether there is an abnormality in a body by using an image in which an inside of the body of a person or an animal is imaged. For example, Patent Documents 1 to 3 disclose a technique of displaying side by side an image (CT image or MRI image) obtained in a past examination (for example, one year ago) and an image obtained in a present examination. Further, Patent Documents 1 and 4 disclose a technique of detecting a lesion from an image and marking the detected lesion.

### RELATED DOCUMENT

### PATENT DOCUMENT

[Patent Document 1] Japanese Patent Application Publication No. 2007-159934
[Patent Document 2] Japanese Patent Application Publication No. 2016-048426
[Patent Document 3] Japanese Patent Application Publication No. 2016-202722
[Patent Document 4] PCT Publication No. WO 2011/132468

### SUMMARY OF THE INVENTION

### TECHNICAL PROBLEM

As one of methods for examining an inside of the body, there is a method to examine a state of the inside of the body by viewing a video displayed on a display device using an endoscope system or the like. Specifically, a doctor inserts a scope that has a camera at a tip from a nose, mouth, anus, or the like of a subject, and moves the scope in the body. By doing so, the state inside the body is imaged by the camera. The doctor checks whether there is an abnormal site in the body of the subject while viewing the state of the inside of the body imaged by the camera using the video displayed on the display device.

As described above, in the method in which the examination is performed by moving the camera in the body of the subject, a site that can be observed by the doctor changes over time since the camera is moved in the body. Therefore, the doctor may miss the abnormal site, and there is actually a difference in the lesion detection rate depending on doctors in charge of the examination. In each related document described above, a situation where the site that can be observed by the doctor changes over time in this manner is not assumed.

The present invention is made in view of the above problems. One of the objects of the present invention is to provide a technique for improving the quality of an examination using a video in which an inside of the body of a subject is imaged.

### SOLUTION TO PROBLEM

The information processing apparatus according to the present invention includes: 1) a detection unit that detects an abnormal region in a body from a video in which the body is imaged; and 2) a display control unit that displays a video frame in which the abnormal region is detected among video frames constituting the video in a first region of a display device and displays the video including the video frame generated after the video frame in a second region of the display device.

A control method according to the present invention is executed by a computer. The control method includes: 1) a detection step of detecting an abnormal region in a body from a video in which the body is imaged; and 2) a display control step of displaying a video frame in which the abnormal region is detected among video frames constituting the video in a first region of a display device and displaying the video including the video frame generated after the video frame in a second region of the display device.

A program according to the present invention causes a computer to execute each step of the control method according to the present invention.

### ADVANTAGEOUS EFFECTS OF INVENTION

According to the present invention, there is provided a technique for improving the accuracy of an examination using a video in which a body of a subject is imaged.

### BRIEF DESCRIPTION OF THE DRAWINGS

The objects described above and other objects, features, and advantages will become more apparent from preferred example embodiments described below and the following drawings accompanying the example embodiments.

Fig. 1 is a diagram conceptually illustrating an operation of an information processing apparatus according to an example embodiment 1.
Fig. 2 is a block diagram illustrating a functional configuration of the information processing apparatus.
Fig. 3 is a diagram illustrating a computer for implementing the information processing apparatus.
Figs. 4 are diagrams illustrating a configuration of a display device.
Fig. 5 is a diagram illustrating a specific example of a usage environment of the information processing apparatus.
Fig. 6 is a flowchart illustrating a flow of processing executed by the information processing apparatus according to the example embodiment 1.
Figs. 7 are diagrams illustrating various superimposition marks superimposed on an abnormal region.
Figs. 8 are diagrams illustrating instruction marks indicating an abnormal region.
Fig. 9 is a diagram illustrating the information processing apparatus connected to an image storage unit.
Fig. 10 is a block diagram illustrating an information processing apparatus according to an example embodiment 2.
Fig. 11 is a diagram illustrating abnormal region information in a table format.
Fig. 12 is a diagram illustrating a scene in which a display on the display device is updated.
Fig. 13 is a diagram illustrating a first display in consideration of a difference in abnormal regions.
Fig. 14 is a diagram illustrating highlighting.
Fig. 15 is a diagram illustrating an example in which the first display is highlighted.
Fig. 16 is a block diagram illustrating an information processing apparatus according to an example embodiment 3.
Fig. 17 is a diagram illustrating a format of information to be stored in the image storage unit in a table format.
Fig. 18 is a block diagram illustrating an information processing apparatus according to an example embodiment 4.
Fig. 19 is a diagram illustrating a scene in which a video frame including an abnormal region targeted for a predetermined action by a user is highlighted.

### DESCRIPTION OF EMBODIMENTS

Hereinafter, example embodiments of the present invention will be described with reference to drawings. Note that, in all the drawings, the same reference numeral is assigned to the same component and the description thereof will not be repeated. In each block diagram, each block represents a configuration of a function unit, not a configuration of a hardware unit, unless otherwise described.

### [Example Embodiment 1]

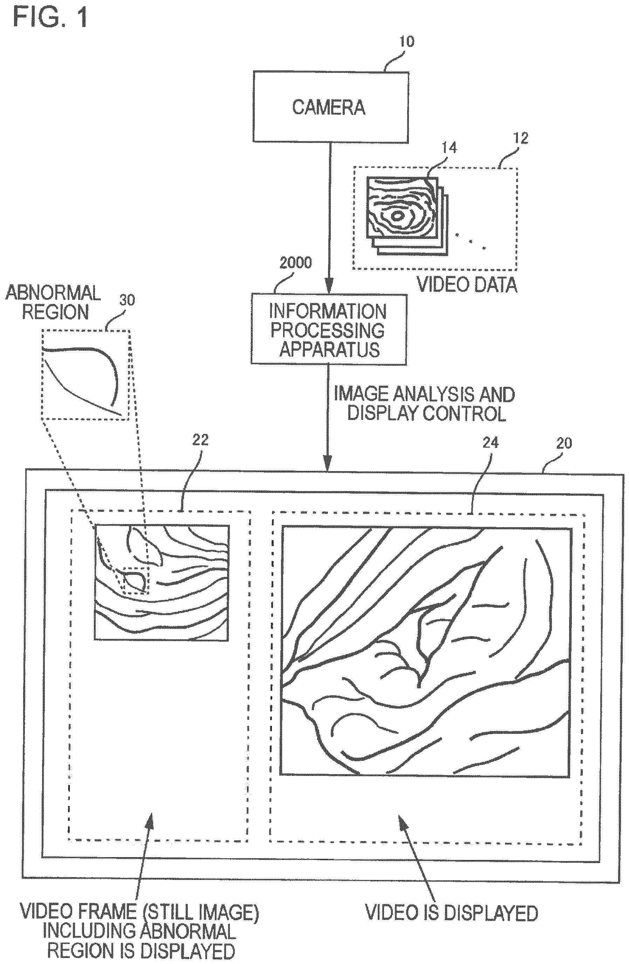

Fig. 1 is a diagram conceptually illustrating an operation of an information processing apparatus 2000 according to an example embodiment 1. Note that Fig. 1 only shows an example of the operation thereof in order to easily understand the information processing apparatus 2000 and does not limit functions of the information processing apparatus 2000.

A camera 10 is used for examining people or other animals. Hereinafter, a person or the like to be examined is referred to as a subject. The camera 10 is any camera capable of imaging an inside of the body of the subject and generates a video frame 14 representing the imaging result. For example, the camera 10 is an endoscope camera. Video data 12 is formed by a plurality of video frames 14 generated at mutually different times.

The video data 12 generated by the camera 10 is displayed on a display device 20. The display device 20 is any display device capable of displaying the video data 12. Note that a fact that the video data 12 is displayed on the display device 20 means that the plurality of video frames 14 constituting the video data 12 are displayed on the display device 20 in order.

A user (for example, doctor) of the information processing apparatus 2000 recognizes a scene in the body of the subject by viewing the video data 12 displayed on the display device 20. More specifically, the user recognizes whether there is an abnormal site in the body of the subject, a degree of the abnormality, or the like. Here, the "abnormal site in the body" is, for example, a site having a lesion, a site having a wound, or a site having a foreign object. The lesion is a change in a living body caused by a disease, such as a tumor.

Here, in an endoscope examination or the like for searching for an abnormal site in the body while the body of the subject is observed by the camera, a doctor may miss the abnormal site even though the abnormal site is imaged by the camera. Therefore, it is preferable to provide support such that the doctor can easily recognize the abnormal site and thus to prevent missing of the abnormal site.

The information processing apparatus 2000 according to the present example embodiment operates as follows. The information processing apparatus 2000 acquires the video data 12 and performs an image analysis of the video frame 14 constituting the video data 12. Specifically, the information processing apparatus 2000 detects an abnormal region 30 from the video frame 14. The abnormal region 30 is a region presumed to represent the abnormal site in the body of the subject. For example, the abnormal region 30 in Fig. 1 is a region including the tumor (region representing the lesion).

The information processing apparatus 2000 displays the video frame 14 from which the abnormal region 30 is detected in a first region 22 of the display device 20, and displays the video data 12 in a second region 24 of the display device 20. In other words, the video frame 14 from which the abnormal region 30 is detected is displayed on the display device 20 together with the video data 12 including the video frames 14 generated thereafter. The first region 22 and the second region 24 are mutually different regions.

For example, the video data 12 generated by the camera 10 is displayed in the second region 24 in real time. That is, the video data 12 to be displayed in the second region 24 represents a scene of the subject at the current timepoint in real time. On the other hand, the video frame 14 generated before the current timepoint in the same examination and imaging the abnormal site in the body is displayed in the first region 22.

In this manner, with the information processing apparatus 2000 according to the present example embodiment, the video frame 14 from which the abnormal region 30 is detected is displayed on the display device 20 together with the video data 12. By doing so, it is possible for the user of the information processing apparatus 2000 to easily recognize the abnormal site in the body of the subject. Even though the user misses a certain abnormal site when the abnormal site is displayed in the second region, the video frame 14 including the abnormal site is displayed and remains in the first region 22 of the display device 20. Therefore, it is possible for the user to recognize the abnormal site later, by browsing the first region 22. As described above, with the information processing apparatus 2000 according to the present example embodiment, it is possible to reduce a probability that the user misses an abnormal site. Accordingly, it is possible to improve the accuracy of the examination of the inside of body performed using the camera 10.

Hereinafter, the present example embodiment will be described in more detail.

### <Functional Configuration>

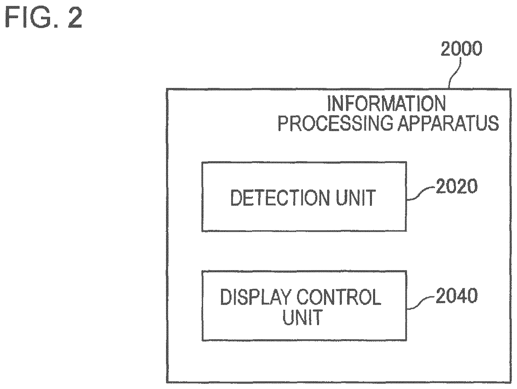

Fig. 2 is a block diagram illustrating a functional configuration of the information processing apparatus 2000. The information processing apparatus 2000 includes a detection unit 2020 and a display control unit 2040. The detection unit 2020 detects the abnormal region 30 from the video data 12. The display control unit 2040 displays the video frame 14 from which the abnormal region 30 is detected in the first region 22 of the display device 20. Further, the display control unit 2040 displays the video data 12 including the video frame 14 generated after the video frame 14 displayed in the first region, on the second region 24 of the display device 20.

### <Example of Hardware Configuration of Information Processing Apparatus 2000>

Each functional configuration unit of the information processing apparatus 2000 may be formed by hardware (for example, a hard-wired electronic circuit or the like) that forms each functional configuration unit or a combination of hardware and software (for example, a combination of an electronic circuit and a program that controls the circuit). Hereinafter, the case where each functional configuration unit of the information processing apparatus 2000 is formed by the combination of hardware and software will be further described.

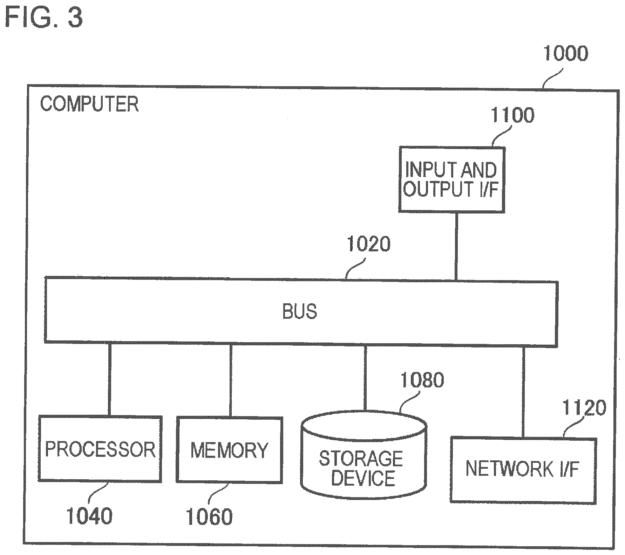

Fig. 3 is a diagram illustrating a computer 1000 for forming the information processing apparatus 2000. The computer 1000 is a variety of computers. For example, the computer 1000 is a personal computer (PC), a server machine, a tablet terminal, a smartphone, or the like. The computer 1000 may be a dedicated computer designed to form the information processing apparatus 2000 or may be a general-purpose computer.

The computer 1000 includes a bus 1020, a processor 1040, a memory 1060, a storage device 1080, an input and output interface 1100, and a network interface 1120. The bus 1020 is a data transmission path for the processor 1040, the memory 1060, the storage device 1080, the input and output interface 1100, and the network interface 1120 to mutually transmit and receive data. The processor 1040 is an arithmetic processing apparatus such as a central processing unit (CPU) or a graphics processing unit (GPU). The memory 1060 is a main storage device formed by a random access memory (RAM) or the like. The storage device 1080 is an auxiliary storage device formed by a hard disk, a solid state drive (SSD), a ROM, or a memory card. However, the storage device 1080 may be formed by hardware similar to the hardware used to form the main storage device, such as the RAM.

The input and output interface 1100 is an interface for connecting the computer 1000 to an input and output device. For example, the camera 10 and the display device 20 are connected to the input and output interface 1100.

The network interface 1120 is an interface for connecting to a communication network such as a wide area network (WAN) or a local area network (LAN).

The storage device 1080 stores a program module that realizes each function of the information processing apparatus 2000. The processor 1040 reads each of the program modules into the memory 1060 and executes each program module to realize each function corresponding to the program module.

### <About Display device 20>

The display device 20 may have one screen or may have a plurality of screens. In the former case, the first region 22 and the second region 24 are different regions from each other on one screen. In the latter case, the first region 22 and the second region 24 may be different regions from each other on one screen, or may be regions on different screens from each other.

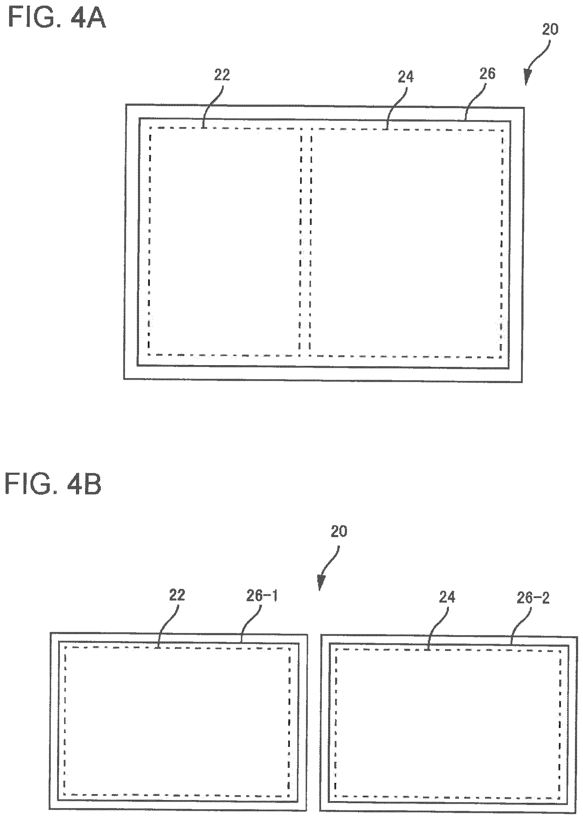

Figs. 4 are diagrams illustrating configurations of the display device 20. The display device 20 in Fig. 4A has one display screen 26. The first region 22 and the second region 24 are different regions from each other on the display screen 26. The display device 20 in Fig. 4B has two display screens 26 (a display screen 26-1 and a display screen 26-2). The first region 22 is the entire region of the display screen 26-1 or a partial region thereof. On the other hand, the second region 24 is the entire region of the display screen 26-2 or a partial region thereof. In the following description, a case where the display device 20 is composed of the one display screen 26 (the case of Fig. 4A) will be described as an example, unless otherwise specified.

### <Specific Example of Usage Environment of Information Processing Apparatus 2000>

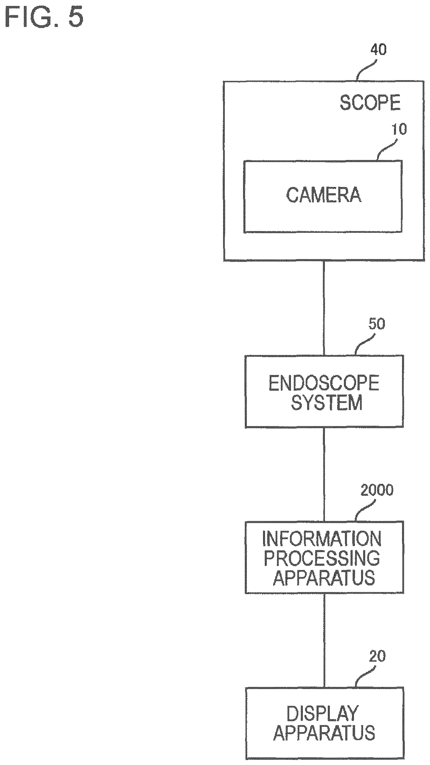

Fig. 5 is a diagram showing a specific example of a usage environment of the information processing apparatus 2000. For example, the information processing apparatus 2000 is used together with a scope 40 and an endoscope system 50. The scope 40 is connected to the endoscope system 50. The scope 40 is provided with the camera 10. In this case, the video data 12 is formed by the plurality of video frames 14 generated by the camera 10 provided in the scope 40. The endoscope system 50 outputs the video data 12 to the information processing apparatus 2000. For example, the video data 12 is output from an interface for video output (for example, High-Definition Multimedia Interface (HDMI) (registered trademark) interface) provided in the endoscope system 50 to an interface for video input of the information processing apparatus 2000. The information processing apparatus 2000 processes the video data 12 acquired from the endoscope system 50 to control the display of the display device 20 (refer to Fig. 1).

Note that the configuration shown in Fig. 5 is merely an example, and the usage environment of the information processing apparatus 2000 is not limited to the configuration shown in Fig. 5. For example, the video data 12 may be output from the camera 10 to the information processing apparatus 2000. In this case, the information processing apparatus 2000 may not be connected to the endoscope system 50.

### <Flow of Processing>

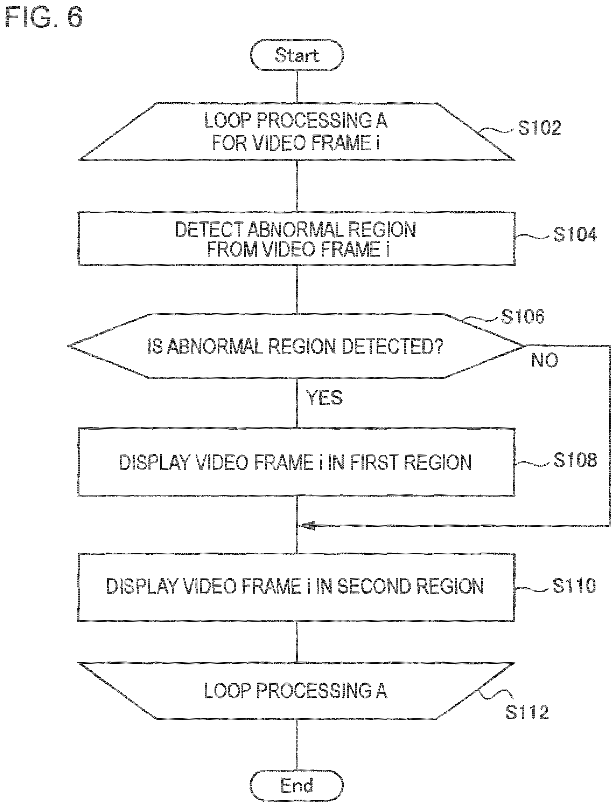

Fig. 6 is a flowchart illustrating a flow of processing executed by the information processing apparatus 2000 according to the example embodiment 1. Steps S102 to S112 are loop processing executed for each video frame 14 acquired from the camera 10. In S102, the information processing apparatus 2000 selects a video frame 14 with the earliest generation timepoint among the video frames 14 not yet subjected to loop processing A. The video frame 14 selected here is denoted as video frame i. Note that in a case where all the video frames 14 are already subjected to the loop processing A, for example, the information processing apparatus 2000 waits until a new video frame 14 is generated. Alternatively, the processing of Fig. 6 may be ended.

The detection unit 2020 detects the abnormal region 30 from the video frame *i* (S104). In a case where the abnormal region 30 is detected from the video frame *i* (YES in S106), the display control unit 2040 displays the video frame *i* in the first region 22 (S108). Accordingly, the video frame 14 from which the abnormal region 30 is detected is displayed in the first region 22.

In S110, the display control unit 2040 displays the video frame *i* in the second region 24. Accordingly, the video frame *i* is displayed in the second region 24 regardless of whether the abnormal region 30 is detected.

Since S112 is the end of the loop processing *A*, the processing of Fig. 6 returns to S102.

Note that the video frame 14 subjected to the processing (S104) of detecting the abnormal region 30 may be all the video frames 14 included in the video data 12 or may be some of the video frames 14. In the latter case, for example, the detection unit 2020 executes S104 only for one video frame 14 for each predetermined number (for example, one for every 10).

### <Acquisition of Video Data 12>

Any method of the detection unit 2020 acquiring the video data 12 may be employed. For example, the detection unit 2020 accesses a storage device in which the video data 12 is stored to acquire the video data 12. The storage device in which the video data 12 is stored may be provided inside the camera 10 or may be provided outside the camera 10. For example, the detection unit 2020 may receive the video data 12 to be transmitted from the camera 10 to acquire the video data 12. Further, the detection unit 2020 may acquire the video data 12 from another apparatus (for example, the above endoscope system 50) connected to the camera 10.

### <Detection of Abnormal Region 30: S104>

The detection unit 2020 detects the abnormal region 30 from each video frame 14 constituting the video data 12. Here, an existing technique can be used as a technique of analyzing an image in which the inside of the body is imaged and detecting an abnormal site. For example, a method such as feature value matching or template matching can be used. For example, in a case where the tumor is detected by the feature value matching, one or more values (feature values) representing a feature of an appearance (color, pattern, shape, or the like) of the tumor are defined in advance. The detection unit 2020 detects, from the video frame 14, an image region with high similarity with a feature value of the tumor set in advance in the image region of the video frame 14. The detection unit 2020 handles the detected image region as an image region representing the abnormal region 30. The same method can be employed for a case where a wound or a foreign object is detected.

Note that in the case where a foreign object is desired to be detected, it is assumed that the foreign object that entered into the body has been determined. In this case, it is preferable to be able to specify a feature value of the foreign object to the information processing apparatus 2000. For example, a photograph of the foreign object that was entered into the body is input to the information processing apparatus 2000. The information processing apparatus 2000 performs the image analysis of the photograph to compute the feature value of the foreign object to be detected. The detection unit 2020 detects the foreign object having the computed feature value from the video frame 14.

### <About First Region 22 and Second Region 24>

As described above, the video frame 14 including the abnormal region 30 is displayed in the first region 22. The video data 12 is displayed in the second region 24. The first region 22 and the second region 24 can be any regions different from each other. Positions and sizes of the first region 22 and the second region 24 in the display device 20 may be fixed or may be not fixed. In the latter case, for example, the display control unit 2040 receives a user operation to change the positions and sizes of the first region 22 and the second region 24. The display control unit 2040 changes the positions and sizes of the first region 22 and the second region 24 in response to the received user operation.

### <Display of Video Frame 14 in First Region 22: S108>

The display control unit 2040 displays the video frame 14 from which the abnormal region 30 is detected in the first region 22 of the display device 20. Here, an existing technique can be used as a technique of displaying an image in an area on the display device 20.

The video frame 14 to be displayed in the first region 22 may be one or plural. In the latter case, for example, the display control unit 2040 displays the video frame 14 from which the abnormal region 30 is detected in the first region 22, in time order from the earlier generation timepoint. Here, there may be a case where the video frame 14 does not fit in the first region 22 since the number of the video frames 14 from which the abnormal region 30 is detected is large. In this case, for example, the display control unit 2040 may change the video frames 14 to be displayed in the first region 22 by displaying a scroll bar or the like on the display device 20.

Note that the video frame 14 from which the abnormal region 30 is detected is displayed on the display device 20 at any timing. For example, the display control unit 2040 displays the video frame 14 on the display device 20 at a timing when the abnormal region 30 is detected from the video frame 14. In another example, the display control unit 2040 displays the video frame 14 on the display device 20 after a predetermined time has elapsed since the abnormal region 30 is detected from the video frame 14. The predetermined time may be set in advance in the display control unit 2040 or may be stored in a storage device accessible from the display control unit 2040.

### <Display of Video Data 12: S110>

The display control unit 2040 displays the video data 12 in the second region 24 of the display device 20. An existing technique can be used as a technique of displaying video data on a display device.

### <Display Representing Abnormal Region 30>

The display control unit 2040 may perform a display representing the abnormal region 30 included in the video frame 14, for the video frame 14 to be displayed in the first region 22. By doing so, it is possible for the user to easily recognize the abnormal region 30 included in the video frame 14. Hereinafter, this display is referred to as a first display.

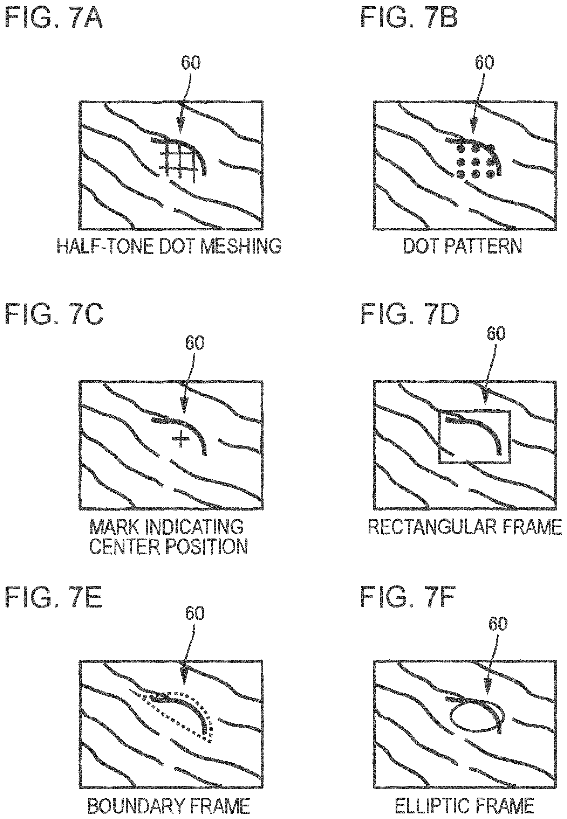

Various displays can be employed as the first display. For example, the display control unit 2040 displays a predetermined mark in the abnormal region 30 so as to be superimposed on the abnormal region 30 of the video frame 14 to be displayed in the first region 22. Hereinafter, this mark is referred to as a superimposition mark. In this example, the superimposition mark is the first display. Figs. 7 are diagrams illustrating various superimposition marks 60 to be superimposed on the abnormal region 30.

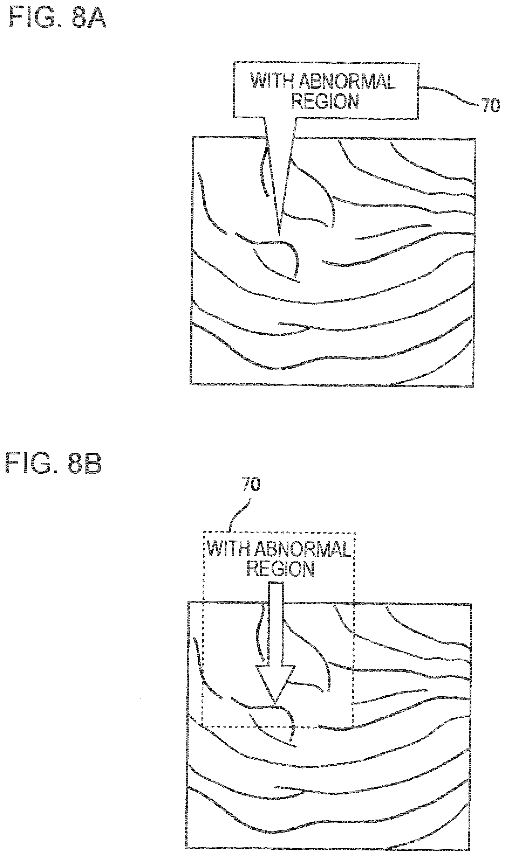

In another example, the display control unit 2040 may perform the first display (hereinafter referred to as an instruction mark) indicating the abnormal region 30 near the video frame 14. Figs. 8 are diagrams illustrating instruction marks 70 indicating the abnormal region 30.

### <Recording of Video Frame 14>

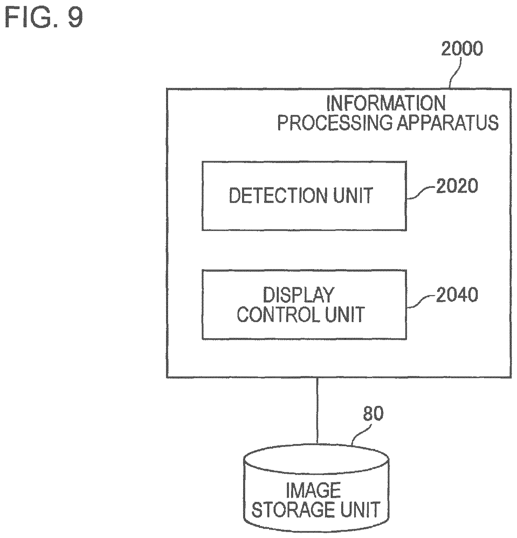

The detection unit 2020 may records the video frame 14 from which the abnormal region 30 is detected in a storage device. Hereinafter, the storage device for storing the video frame 14 is referred to as an image storage unit 80. Fig. 9 is a diagram illustrating the information processing apparatus 2000 connected to the image storage unit 80. Note that the image storage unit 80 may be provided inside the information processing apparatus 2000.

The detection unit 2020 may record the video frame 14 from which the abnormal region 30 is detected in the image storage unit 80 as it is, or may process the video frame 14 as appropriate and records the processed video frame 14 in the image storage unit 80. For example, the detection unit 2020 records a video frame 14 on which an image (superimposition mark 60 or the like) indicating a position of the abnormal region 30 is superimposed, in the image storage unit 80. By doing so, it is possible to easily recognize the position of the abnormal region 30 in the video frame 14. In another example, the detection unit 2020 records the video frame 14 in the image storage unit 80 in association with information determining the position of the abnormal region 30 included in the video frame 14.

### [Example Embodiment 2]

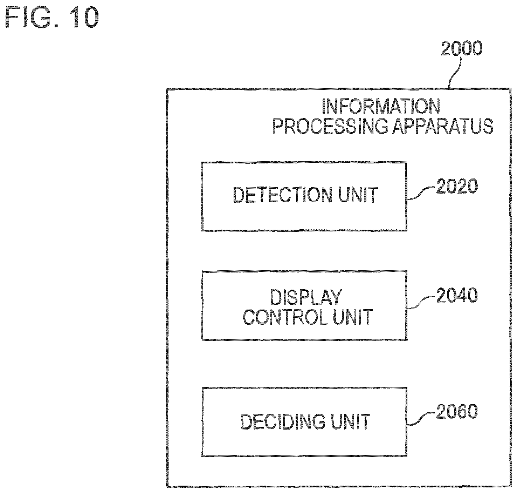

Fig. 10 is a block diagram illustrating an information processing apparatus 2000 according to an example embodiment 2. The information processing apparatus 2000 according to the example embodiment 2 is the same as the information processing apparatus 2000 according to the example embodiment 1 except for the matters described below.

In general, a camera generates video frames at a frequency such as 30 frames per second (fps). Therefore, the plurality of video frames 14 may include the same site. For example, when a certain abnormal region 30 is within an imaging range of the camera 10 for one second, the abnormal region 30 is detected from 30 video frames 14 at maximum. As described above, in the case where the same abnormal region 30 is detected from the plurality of video frames 14, it is not necessarily required to display all of the plurality of video frames 14 in the first region 22. Only by displaying some (for example, one) of the plurality of video frames 14 in the first region 22, the user can recognize the abnormal region 30 included in the video frame 14.

In the case where there are the plurality of video frames 14 including the same abnormal region 30 as described above, the information processing apparatus 2000 according to the example embodiment 2 displays only some of the video frames 14 on the display device 20. For this purpose, the information processing apparatus 2000 according to the example embodiment 2 includes a deciding unit 2060. The deciding unit 2060 decides whether the abnormal region 30 detected from each of the plurality of video frames 14 is the same. The display control unit 2040 according to the example embodiment 2 displays, on the display device 20, only some video frames 14 among the plurality of video frames 14 including the abnormal regions 30 decided to be the same.

### <Decision by Deciding Unit 2060>

The deciding unit 2060 compares the abnormal regions 30 detected from the video frames 14 to decide whether the abnormal regions 30 detected from the video frames 14 are the same. For example, the deciding unit 2060 computes the similarity between the image area representing the abnormal region 30 included in a certain video frame 14 and the image area representing the abnormal region 30 included in another video frame 14. When the similarity is equal to or larger than a predetermined value, the deciding unit 2060 decides that the abnormal regions 30 included in the two video frames 14 are the same. On the other hand, when the similarity is equal to or larger than the predetermined value, the deciding unit 2060 decides that the abnormal regions 30 included in the two video frames 14 are different from each other. Here, an existing technique can be used as a technique of computing similarity by comparing image regions.

Note that the deciding unit 2060 may compare image regions having a predetermined size or shape including the abnormal region 30 and therearound, for the plurality of video frames 14. In another example, the deciding unit 2060 may compare image areas around the abnormal region 30 instead of the abnormal region 30, for the plurality of video frames 14.

A more specific example of the method of deciding whether the abnormal regions 30 included in each video frame 14 are the same will be described. When the abnormal region 30 is detected from the video frame 14, the detection unit 2020 computes the feature value of an image region representing the abnormal region 30 (for example, a parameter representing a shape or pattern of the image region). The detection unit 2020 records the computed feature value in the storage device in association with a discriminator (for example, frame number) of the video frame 14. This storage device can be handled as a database in which information for managing the abnormal region 30 detected from the video data 12 is stored. Hereinafter, information to be stored in the storage device is referred to as abnormal region information.

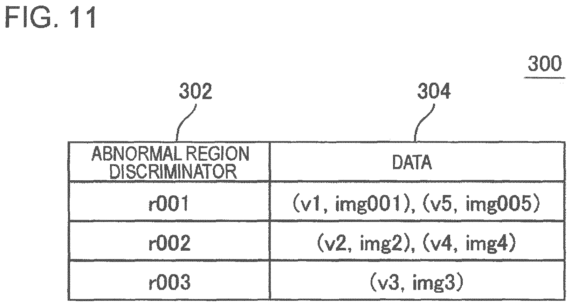

Fig. 11 is a diagram illustrating the abnormal region information in a table format. The table shown in Fig. 11 is referred to as a table 300. The table 300 has two columns of an abnormal region discriminator 302 and data 304. The abnormal region discriminator 302 is a discriminator assigned to the abnormal region 30. The data 304 indicates a set of "feature value of an abnormal region 30 and discriminator of a video frame 14 from which the abnormal region 30 is detected". For example, a record in a first row of the table 300 represents that the abnormal region 30 having a discriminator *r1* is detected from the video frame 14 having a discriminator *img001* and the video frame 14 having a discriminator *img004.* Further, the record in the first row of the table 300 represents that the feature value of the abnormal region 30 detected from the video frame 14 having the discriminator *img001* is *v1*, and the feature value of the abnormal region 30 detected from the video frame 14 having the discriminator *img004* is v5. Since the similarity between the feature values *v1* and *v5* is high, (*v1, img001*) and (v5, *img005*) are stored in the same record.

When the abnormal region 30 is detected from the video frame 14, the detection unit 2020 adds a set of "feature value of the abnormal region 30 and discriminator of the video frame 14" to the table 300. In this case, the deciding unit 2060 searches for a feature value having the high similarity with the feature value of the detected abnormal region 30 from the table 300. It is assumed that, as a result of the search, a record indicating a feature value having the high similarity with the feature value thereof is found in the data 304. In this case, the deciding unit 2060 updates the record obtained by the search to add the feature value of the detected abnormal region 30 and the discriminator of the video frame 14 from which the abnormal region 30 is detected, to the table 300. Specifically, the deciding unit 2060 adds a set of "feature value of the detected abnormal region 30 and the discriminator of the video frame 14 from which the abnormal region 30 is detected" to the data 304 of the record obtained by the search.

On the other hand, it is assumed that, as a result of the search, a record indicating a feature value having the high similarity with the feature value of the detected abnormal region 30 is not found in the data 304. In this case, the deciding unit 2060 generates a new record indicating "feature value of the detected abnormal region 30 and discriminator of the video frame 14 from which the abnormal region 30 is detected", and adds the record to the table 300.

Note that in a case where a plurality of abnormal regions 30 are detected from one video frame 14, the above processing is performed for each of the plurality of abnormal regions 30.

It is possible to easily decide whether the same abnormal region 30 is included in a plurality of video frames 14 by managing the abnormal regions 30 detected from each video frame 14 in this manner. Specifically, when a record indicating a discriminator of a certain video frame 14 is the same as a record indicating a discriminator of another video frame 14 in the table 300, the deciding unit 2060 decides that the same abnormal region 30 is included in those video frames 14. On the other hand, when a record indicating a discriminator of a certain video frame 14 is different from a record indicating a discriminator of another video frame 14 in the table 300, the deciding unit 2060 decides that mutually different abnormal regions 30 are included in those video frames 14.

### <Method of Deciding Video Frame 14 to be Displayed on Display device 20>

For example, the display control unit 2040 determines one video frame 14 in which the abnormal region 30 is most easily recognized by the user among the plurality of video frames 14 including the abnormal region 30 decided to be the same, and displays the determined video frame 14 on the display device 20. Various methods can be employed for the above determination. Hereinafter, specific examples of the above determination method will be described.

### <<Method of Using Likelihood Representing Abnormality>>

The display control unit 2040 determines likelihood with which an image region representing the abnormal region 30 represents an abnormality in the body, for the plurality of video frames 14 including the abnormal regions 30 decided to be the same. For example, in a case where the abnormal region 30 is detected from the video frame 14 by the feature value matching or the template matching, the likelihood with which the image region representing the abnormal region 30 represents the abnormality in the body is represented by a degree of similarity between the image region and a feature value or a template defined in advance. The display control unit 2040 determines a video frame 14 with the highest likelihood as the video frame 14 to be displayed on the display device 20.

It can be consider that the higher the likelihood of that the abnormal region 30 included in the video frame 14 represents the abnormality in the body is, the more clearly the abnormal region 30 included in the video frame 14 represents the abnormality. Therefore, it enables the user to recognize the abnormality in the body of the subject more precisely by displaying, on the display device 20, the video frame 14 with high likelihood of that the abnormal region represents the abnormality in the body.

### <<Method of Using Position of Abnormal Region 30>>

The display control unit 2040 determines a video frame 14 in which a position of the abnormal region 30 is closest to the center position of the video frame 14 among the plurality of video frames 14 including the abnormal region 30 decided to be the same, and handles the determined video frame 14 as the video frame 14 to be displayed on the display device 20. Specifically, the display control unit 2040 computes, for each video frame 14, a distance between the abnormal region 30 included in the video frame 14 and the center coordinate of the video frame 14. The display control unit 2040 determines a video frame 14 having the smallest distance as the video frame 14 to be displayed on the display device 20.

In general, an object included in the image generated by a camera is easier to be seen as the object is near the center of the image. Therefore, it becomes easier for the user to see the abnormal region 30 by displaying, on the display device 20, the video frame 14 in which the position of the abnormal region 30 is close to the center position of the video frame 14.

### <<Method of Using Contrast of Entire Video Frame 14>>

The display control unit 2040 determines a video frame 14 having the highest contrast in the entire video frame 14 among the plurality of video frames 14 including the abnormal regions 30 decided to be the same as the video frame 14 to be displayed on the display device 20. Specifically, the display control unit 2040 computes an index value representing the contrast of the entire video frame 14 for each video frame 14. The display control unit 2040 compares the computed index values to determine the video frame 14 having the highest contrast, and handles the determined video frame 14 as the video frame 14 to be displayed on the display device 20. Note that, for example, Michelson contrast or the like can be used for the index value representing the contrast.

In general, it is easier to distinguish individual objects included in an image as the contrast of the image is higher. Therefore, it becomes easier for the user to see the abnormal region by displaying, on the display device 20, the video frame 14 having high contrast in the entire video frame 14.

### <<Method of Using Contrast of Image Region Representing Abnormal Region 30>>

The display control unit 2040 may use the contrast of the image region representing the abnormal region 30 instead of the contrast of the entire video frame 14. That is, the display control unit 2040 computes the index value of the contrast of the image area representing the abnormal region 30 for each of the plurality of video frames 14 including the abnormal region 30 decided to be the same. The display control unit 2040 compares the computed index values to determine the video frame 14 having the highest contrast in the image region representing the abnormal region 30, and displays the determined video frame 14 on the display device 20.

With this method, it is easier for the user to see the inside of the abnormal region 30 since the abnormal region 30 having the high contrast is displayed on the display device 20.

### <Timing of Displaying Video Frame 14 on Display device 20>

As described above, the video frame 14 from which the abnormal region 30 is detected is displayed on the display device 20 at any timing. For example, the display control unit 2040 displays the video frame 14 on the display device 20 at the timing when the abnormal region 30 is detected from the video frame 14. In this case, for example, the display control unit 2040 compares a video frame 14 already displayed on the display device 20 with a new video frame 14 in which the abnormal region 30 that is the same as the abnormal region 30 included in the video frame 14 is detected, in order to decide the video frame 14 to be displayed on the display device 20. In a case where the new video frame 14 is decided to be displayed on the display device 20, the display is updated on the display device 20. On the other hand, in a case where the video frame 14 already displayed in the video frame 14 is decided to be displayed on the display device 20, the display control unit 2040 does not display the new video frame 14 on the display device 20.

For example, the display control unit 2040 compares the likelihood of that the abnormal region 30 included in the video frame 14 already displayed on the display device 20 represents the abnormality with the likelihood of that the abnormal region 30 included in the new video frame 14 represents the abnormality. In a case where the abnormal region 30 included in the new video frame 14 has higher likelihood of representing the abnormality, the display control unit 2040 updates the display on the display device 20 to display the new video frame 14 on the display device 20. On the other hand, in a case where the abnormal region 30 included in the video frame 14 already displayed on the display device 20 has higher likelihood of representing the abnormality, the display control unit 2040 does not update the display on the display device 20.

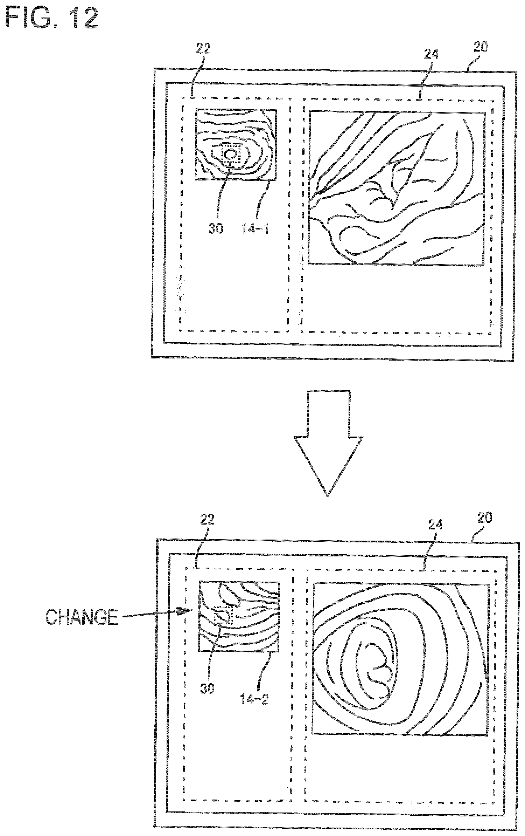

Fig. 12 is a diagram illustrating a scene in which the display of the display device 20 is updated. In the display device 20 on the upper part, a video frame 14-1 is displayed in the first region 22. Thereafter, it is assumed that the same abnormal region 30 as the abnormal region 30 included in the video frame 14-1 is detected from a video frame 14-2, which is generated after the video frame 14-1. Further, it is assumed that the likelihood of that the abnormal region 30 included in the video frame 14-2 represents the abnormality is higher than the likelihood of that the abnormal region 30 included in the video frame 14-1 represents the abnormality.

In this case, the display control unit 2040 changes the video frame 14 to be displayed in the first region 22 from the video frame 14-1 to the video frame 14-2 (See the lower part of Fig. 12). On the other hand, in a case where the likelihood of that the abnormal region 30 included in the video frame 14-2 represents the abnormality is lower than the likelihood of that the abnormal region 30 included in the video frame 14-1 represents the abnormality, the display control unit 2040 does not change the video frame 14 to be displayed in the first region 22 (not shown).

The timing when the display control unit 2040 displays the video frame 14 on the display device 20 is not limited to the timing described above. For example, the display control unit 2040 does not display the video frame 14 on the display device 20 while the same abnormal region 30 is detected from the plurality of video frames 14 that are continuous in a time series. After the same abnormal region 30 is no longer detected from the video frame 14, the display control unit 2040 determines one video frame 14 to be displayed on the display device 20 among the plurality of video frames 14 in which the abnormal region 30 has been detected so far. The display control unit 2040 displays the determined video frame 14 on the display device 20.

### <About Storing in Image Storage Unit 80 of Video Frame 14 >

The detection unit 2020 according to the example embodiment 2 may records only some video frames 14 in the image storage unit 80 among the video frames 14 from which the same abnormal region 30 is detected. For example, the detection unit 2020 records, in the image storage unit 80, only the video frame 14 (video frame 14 determined by each method described above) to be displayed in the first region 22 by the detection unit 2020 among the video frames 14 in which the same abnormal region 30 is detected. By doing so, it is possible to save the storage area of the image storage unit 80 while the video frame 14 that well represents the abnormal region 30 is stored.

Here, the detection unit 2020 records the video frame 14 in the image storage unit 80 at any time. For example, the detection unit 2020 records the video frame 14 in the image storage unit 80 at the timing when the abnormal region 30 is detected from the video frame 14. In this case, the detection unit 2020 compares a video frame 14 already stored in the image storage unit 80 with a new video frame 14 in which the abnormal region 30 that is the same as the abnormal region 30 included in the video frame 14 is detected, in order to decide the video frame 14 to be stored in the image storage unit 80. In a case where the new video frame 14 is decided to be stored in the image storage unit 80, the detection unit 2020 deletes the video frame 14 already stored in the image storage unit 80 and records the new video frame 14 in the image storage unit 80. On the other hand, in a case where the video frame 14 already stored in the image storage unit 80 is decided to be stored in the image storage unit 80, the detection unit 2020 does not records the new video frame 14 in the image storage unit 80.

In another example, the detection unit 2020 does not records the video frame 14 in the image storage unit 80 while the same abnormal region 30 is detected from the plurality of video frames 14 that are continuous in a time series. After the same abnormal region 30 is no longer detected from the video frame 14, the detection unit 2020 determines one video frame 14 to be stored in the image storage unit 80 among the plurality of video frames 14 in which the abnormal region 30 has been detected so far. The detection unit 2020 records the determined video frame 14 in the image storage unit 80.

### <About First Display>

As described above, the display control unit 2040 may display the first display representing the abnormal region 30 on the display device 20. In this case, it is preferable that the same first display is used for the same abnormal region 30, and the mutually different abnormal regions 30 are used for the different abnormal regions 30. By doing so, it is possible to easily distinguish whether the abnormal region 30 included in each of the plurality of video frames 14 displayed on the display device 20 is the same. Therefore, the examination using the information processing apparatus 2000 can be performed more smoothly.

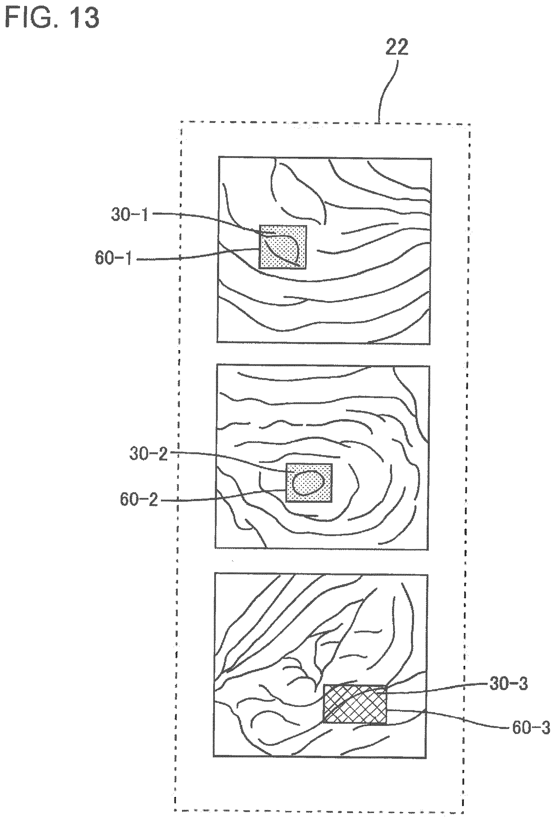

There are various methods of making the first display different for each of the mutually different abnormal regions 30. For example, the display control unit 2040 uses the first display having the same color or shape for the same abnormal region 30, and uses the first display having a different colors or shapes for the mutually different abnormal regions 30. Fig. 13 is a diagram illustrating a first display in consideration of a difference in abnormal regions 30. In Fig. 13A, an abnormal region 30-1 included in a video frame 14-1 and an abnormal region 30-2 included in a video frame 14-2 are the same abnormal region 30. On the other hand, an abnormal region 30-3 included in a video frame 14-3 is an abnormal region 30 different from the abnormal region 30-1 and the abnormal region 30-2. The display control unit 2040 displays a superimposition mark 60-1 and a superimposition mark 60-2 having the same pattern (dot pattern) respectively on the abnormal region 30-1 and the abnormal region 30-2. On the other hand, the display control unit 2040 displays a superimposition mark 60-3 having a lattice pattern different from the dot pattern on the abnormal region 30-3.

### <Abnormal Region 30 to be Displayed in Both First Region 22 and Second Region 24>

The user of the information processing apparatus 2000 performs the examination while moving the camera 10 in the body of the subject. Therefore, the abnormal region 30 going out of the imaging range of the camera 10 once may enter the imaging range of the camera 10 again. For example, it is assumed that a doctor views a video frame 14 displayed in the first region 22 and recognizes that the doctor misses the abnormal region 30 included in the video frame 14 (did not view that abnormal region 30 when that abnormal region 30 was displayed in the second region 24 as a video). In this case, there may be a case where the doctor operates the camera 10 (for example, operate the scope 40) such that the abnormal region 30 falls in the imaging range of the camera 10 again in order to recognize the details by viewing the abnormal region 30 with the video. As a result, the same abnormal region 30 is displayed in the first region 22 and the second region 24. In other words, the same abnormal region 30 is displayed in the video frame representing a scene in the past and the video representing a real-time scene.

In the case where the same abnormal region 30 is displayed in the first region 22 and the second region 24 in this manner, it is preferable that the display control unit 2040 notifies the fact. For example, in a case where the abnormal region 30 is detected from the video frame 14 to be displayed in the second region 24, the display control unit 2040 decides whether the video frame 14 including that abnormal region 30 is displayed in the first region 22. In a case where the video frame 14 including that abnormal region 30 is displayed in the first region 22, the display control unit 2040 performs a predetermined notification.

Any notification method may be employed. For example, the display control unit 2040 may perform highlighting of the video frame 14 including the same abnormal region 30 as the abnormal region 30 detected from the video frame 14 to be displayed in the second region 24, among the video frames 14 displayed in the first region 22.

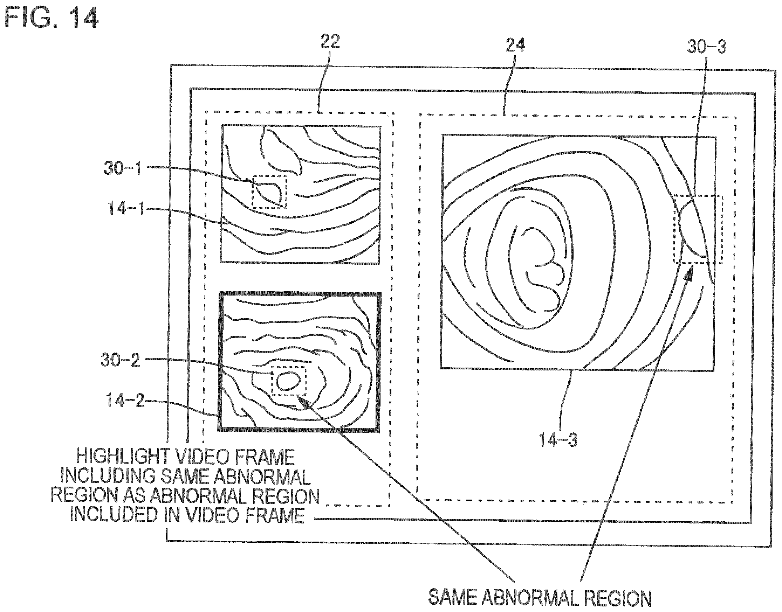

Fig. 14 is a diagram illustrating highlighting. In Fig. 14, the two video frames 14 (video frame 14-1 and video frame 14-2) are displayed in the first region 22. The abnormal region 30-1 and the abnormal region 30-2 are detected from the video frame 14-1 and the video frame 14-2, respectively. These are mutually different abnormal regions 30. The abnormal region 30-3 is detected from the video frame 14-3 which is the current frame of the video data 12 displayed in the second region 24. The abnormal region 30-2 and the abnormal region 30-3 represent the same abnormal region 30.

In this case, the display control unit 2040 highlights the video frame 14-2 including the same abnormal region 30 as the abnormal region 30 included in the video frame 14-3. In Fig. 14, the frame line of the video frame 14-2 is thickened to highlight the video frame 14-2. By doing so, the doctor can easily recognize that the abnormal region 30 (abnormal region 30 displayed in the second region 24) being imaged in real time by the camera 10 is the same as the abnormal region 30 included in the video frame 14-2 among the abnormal regions 30 imaged in the past.

Note that the method of highlighting the video frame 14 is not limited to the method of "thickening the frame line of the video frame 14" shown in the example of Fig. 14. For example, various methods such as a method of blinking the video frame 14 or a method of changing the color of the video frame 14 can be employed.

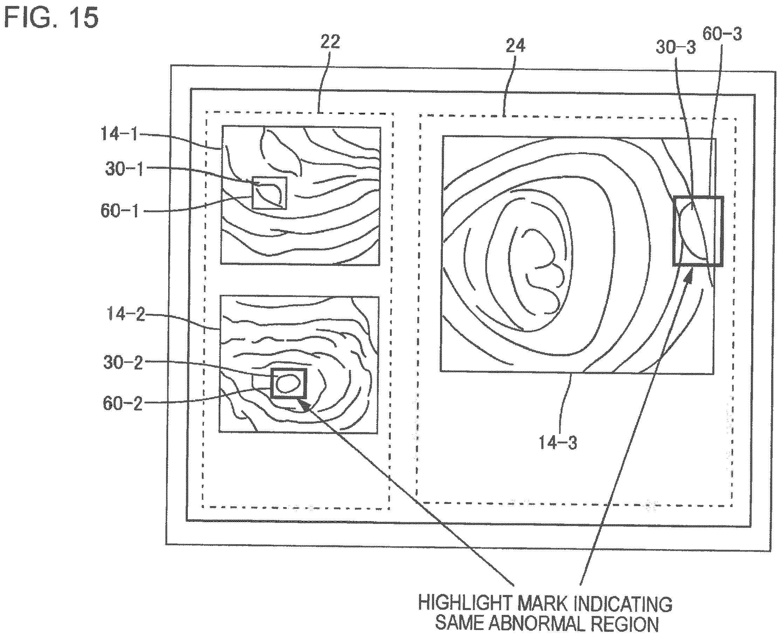

The display control unit 2040 may highlight the first display indicating the abnormal region 30 displayed in the first region 22 and the first display indicating the abnormal region 30 displayed in the second region 24. By doing this, the user of the information processing apparatus 2000 can easily recognize where the same abnormal region 30 as the abnormal region 30 included in the video is included in the past video frame. Fig. 15 is a diagram illustrating an example in which the first display is highlighted. Fig. 15 represents the same situation as Fig. 14 except that the first display is highlighted. In Fig. 15, the superimposition mark 60 is displayed on the abnormal region 30.

In Fig. 15, similarly to Fig. 14, the abnormal region 30-2 included in the video frame 14-2 and the abnormal region 30 included in the video frame 14-3 are the same abnormal region 30. The display control unit 2040 thickens the frame lines of the superimposition mark 60 indicating these two abnormal regions 30. By doing so, the user of the information processing apparatus 2000 can easily recognize that the abnormal region 30-1 indicated by the superimposition mark 60-1 and the abnormal region 30-2 indicated by the superimposition mark 60-2 are the same.

Note that, when the abnormal region 30 to be displayed on the first region 22 and the abnormal region 30 to be displayed on the second region 24 are the same, it may be allowed to display the same first display for those abnormal regions. For example, the superimposition marks 60-1 and 60-2 in Fig. 15 have the same shape. By doing this, it is possible to easily recognize whether the abnormal region 30 included in the real-time video is the same as the abnormal region 30 included in the video frame in the past.

The method of notifying that the same abnormal region 30 is displayed in the first region 22 and the second region 24 is not limited to the highlighting described above. For example, this notification may be an output of a predetermined sound such as a beep sound. In another example, this notification may be a notification that outputs a predetermined vibration.

### <Hardware Configuration>

A hardware configuration of a computer that forms the information processing apparatus 2000 according to the example embodiment 2 is represented, for example, by Fig. 3 similarly to the example embodiment 1. However, the storage device 1080 of the computer 1000 that forms the information processing apparatus 2000 according to the present example embodiment further stores a program module that realizes the functions of the information processing apparatus 2000 according to the present example embodiment.

### [Example Embodiment 3]

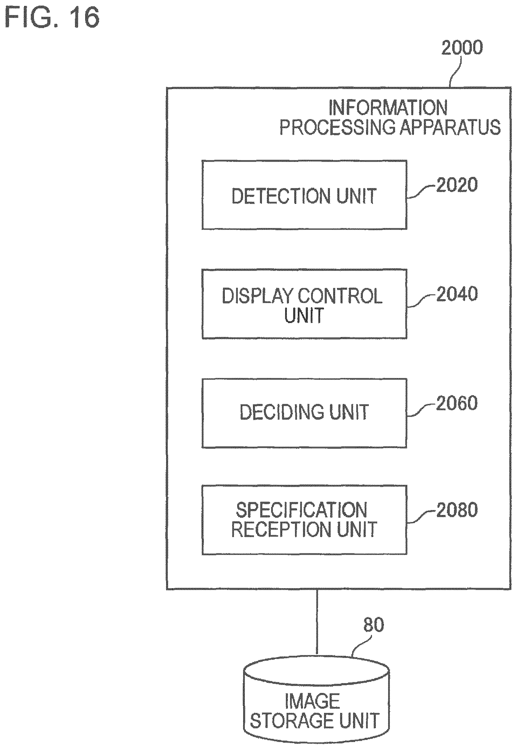

Fig. 16 is a block diagram illustrating an information processing apparatus 2000 according to an example embodiment 3. The information processing apparatus 2000 according to the example embodiment 3 is the same as the information processing apparatus 2000 according to the example embodiment 1 or 2 except for the matters described below.

The information processing apparatus 2000 according to the example embodiment 3 has a specification reception unit 2080. The deciding unit 2060 receives an input specifying one of the plurality of video frames 14 constituting the video data 12 from the user. The deciding unit 2060 records the specified video frame 14 in the image storage unit 80. The detection unit 2020 according to the third embodiment stores the video frame 14 from which the abnormal region 30 is detected in the image storage unit 80.

Here, the detection unit 2020 records the video frame 14 from which the abnormal region 30 is detected so as to be discriminable from the video frame 14 to be recorded in the image storage unit 80 by the specification reception unit 2080 (video frame 14 specified to the specification reception unit 2080). In other words, the video frame 14 to be recorded in the image storage unit 80 by the detection unit 2020 and the video frame 14 to be recorded in the image storage unit 80 by the specification reception unit 2080 are recorded so as to be discriminable from each other. Hereinafter, the video frame 14 to be recorded in the image storage unit 80 by the detection unit 2020 is referred to as an automatic storage frame. Further, the video frame 14 to be recorded in the image storage unit 80 by the specification reception unit 2080 is referred to as an automatic storage frame.

The specification with respect to the specification reception unit 2080 is performed by, for example, a doctor who performs the examination. For example, when the doctor finds an abnormal site during the examination while viewing the video data 12 displayed in the second region 24, the doctor tries to record the video frame 14 including the site. In another example, the doctor may record a video frame 14 including a predetermined site to be noted regardless of whether the site is abnormal.

In such a case, for example, the doctor operates an input device, such as a keyboard or a predetermined button, in a situation where the site is included in the video frame 14 displayed in the second region 24 in order to specify the video frame 14 including the site. The specification reception unit 2080 records the specified video frame 14 in this manner in the image storage unit 80. From the viewpoint of the user, the above operation is, for example, an operation of capturing a photograph by releasing the shutter of the camera.

On the contrary, the automatic storage frame to be recorded in the image storage unit 80 by the detection unit 2020 is a video frame 14 including an abnormal region 30 automatically detected by the image analysis by the information processing apparatus 2000, not specified by the user. That is, the automatic storage frame is the video frame 14 automatically recorded in the image storage unit 80 by the information processing apparatus 2000.

As described above, the specified storage frame and the automatic storage frame are different in triggers of being recorded in the image storage unit 80 and meanings of the frames for the user. Accordingly, it is preferable that the user can easily distinguish whether the video frame 14 stored in the image storage unit 80 is the specified storage frame or the automatic storage frame.

In this point, with the information processing apparatus 2000 according to the present example embodiment, the video frame 14 to be recorded in the image storage unit 80 by the detection unit 2020 and the video frame 14 to be recorded in the image storage unit 80 by the specification reception unit 2080 are recorded in the image storage unit 80 so as to be discriminable from each other. Accordingly, it is possible to easily distinguish whether the video frame 14 stored in the image storage unit 80 is the specified storage frame or the automatic storage frame.

### <Discrimination Method>

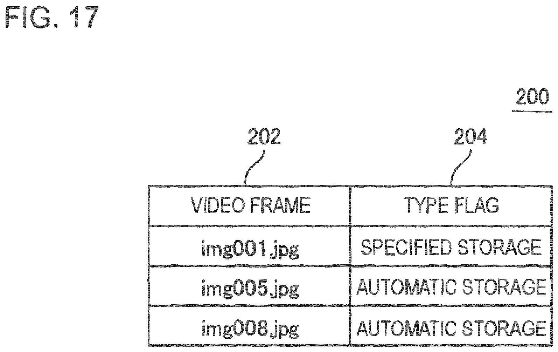

Any method of storing the specified storage frame and the automatic storage frame in the image storage unit 80 in a discriminable manner may be employed. For example, the information processing apparatus 2000 records a flag representing whether the video frame 14 is the specified storage frame or the automatic storage frame in the image storage unit 80 in association with the video frame 14. Fig. 17 is a diagram illustrating a format of information to be stored in the image storage unit 80 in a table format. The table shown in Fig. 17 is denoted by a table 200. The table 200 has two columns of a video frame 202 and a type flag 204. The video frame 202 represents the video frame 14 itself. The type flag 204 represents whether the video frame 14 shown in the video frame 202 is the specified storage frame or the automatic storage frame.

### <About Display by Display Control Unit 2040>

In a case where there is a video frame 14 including the same abnormal region 30 as the abnormal region 30 included in the specified storage frame among the video frames 14 to be displayed in the first region 22, the display control unit 2040 makes the video frame 14 discriminable from the other video frames 14. For example, the display control unit 2040 performs a predetermined display in the video frame 14 including the same abnormal region 30 as the abnormal region 30 included in the specified storage frame or the periphery of the video frame 14. This predetermined display is denoted by a second display. For example, the second display highlights the video frame 14 including the same abnormal region 30 as the abnormal region 30 included in the specified storage frame. Note that the method described in the example embodiment 2 can be used as the method of highlighting a determined video frame 14.

One of the purposes of displaying the video frame 14 from which the abnormal region 30 is detected in the first region 22 is to prevent the user from missing the abnormal region 30. In this point, it can be that the abnormal region 30 included in the specified storage frame, which is the video frame 14 specified by the user, is noticed by the user and is not missed by the user.

In a case where the video frame 14 including the same abnormal region 30 as the abnormal region 30 included in the specified storage frame is displayed in the first region 22, the detection unit 2020 enables the user to recognize the video frame 14. By doing this, it is possible for the user to easily recognize a fact that the user has recognized the abnormal region 30 displayed in the first region 22.

However, the display control unit 2040 may perform the second display in the video frame 14 including the abnormal region 30 different from the abnormal region 30 included in the specified storage frame, and may not perform the second display in the video frame 14 including the same abnormal region 30 as the abnormal region 30 included in the specified storage frame, among the video frames 14 to be displayed in the first region 22. By doing this, the video frame 14 including the abnormal region 30 with a high probability of not being recognized by the user is highlighted among the abnormal regions 30 automatically detected by the information processing apparatus 2000. Accordingly, it is possible for the user to easily recognize the abnormal region 30 that was not recognized by the user.

In another example, the display control unit 2040 may not display the video frame 14 including the same abnormal region 30 as the abnormal region 30 included in the specified storage frame in the first region 22 (delete from the first region 22) among the video frames 14 in which the abnormal regions 30 are detected by the detection unit 2020. By doing this, in the first region 22, only the abnormal region 30 with a high probability of not being recognized by the user is displayed on the display device 20 among the video frames 14 in which the abnormal regions 30 are detected by the detection unit 2020. Accordingly, it is possible for the user to easily recognize the abnormal region 30 that was not recognized by the user.

### <Example of Hardware Configuration>

A hardware configuration of a computer that forms the information processing apparatus 2000 according to the example embodiment 3 is represented, for example, by Fig. 3 similar to the example embodiment 1. However, the storage device 1080 of the computer 1000 that forms the information processing apparatus 2000 according to the present example embodiment further stores a program module that realizes the functions of the information processing apparatus 2000 according to the present example embodiment.

### [Example Embodiment 4]

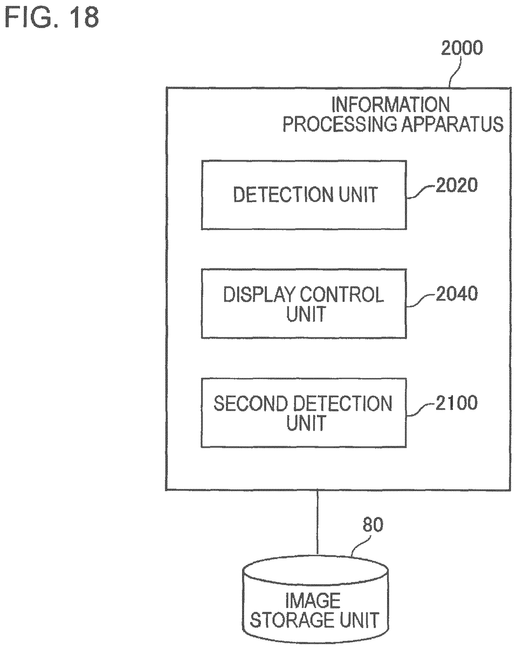

Fig. 18 is a block diagram illustrating an information processing apparatus 2000 according to an example embodiment 4. The information processing apparatus 2000 according to the example embodiment 4 is the same as the information processing apparatus 2000 according to the example embodiment 1, 2, or 3 except for the matters described below.

The information processing apparatus 2000 according to the example embodiment 4 includes a second detection unit 2100. The second detection unit 2100 detects a predetermined action by the user to the abnormal region 30 or the periphery thereof. When the user who examines the inside of the body of the subject finds a site that is likely to be abnormal, the user performs various actions to observe the site in more detail. Examples of the action are 1) changing a color or intensity of light irradiated to the abnormal region 30 or the periphery thereof, 2) performing dye spraying or coloring in the abnormal region 30 or the periphery thereof, 3) administering water or a medicine to the abnormal region 30 or the periphery thereof, and 4) collecting a tissue of the abnormal region 30 or the periphery thereof. It can be that the abnormal region 30 targeted for these actions is a site having a high probability of being recognized by the user, similarly to the abnormal region 30 specified by the user operation with respect to the specification reception unit 2080. Here, the "abnormal region 30 targeted for the predetermined action" means the abnormal region 30 in which the predetermined action by the user with respect to the abnormal region 30 or the periphery of the abnormal region 30 is detected by the second detection unit 2100.

For example, the display control unit 2040 performs the same control as the display control (refer to the example embodiment 3) performed on the same abnormal region 30 as the abnormal region 30 included in the specified storage frame, on the abnormal region 30 targeted for the predetermined action by the user. More specifically, for example, the display control unit 2040 performs: displaying a predetermined display on the abnormal region 30, which is targeted for the predetermined action by the user and displayed in the first region 22, or on the periphery thereof; or displaying a predetermined display in the video frame 14 including the abnormal region 30 or its periphery. The predetermined display is referred to as a third display. The third display is a display, for example, highlighting the abnormal region 30 or the video frame 14. By doing this, it is possible for the user to easily recognize, among the abnormal regions 30 displayed in the first region 22, the abnormal region 30 that has been recognized by the user. Note that the various displays described above can be used to highlight the determined abnormal region 30 or video frame 14.

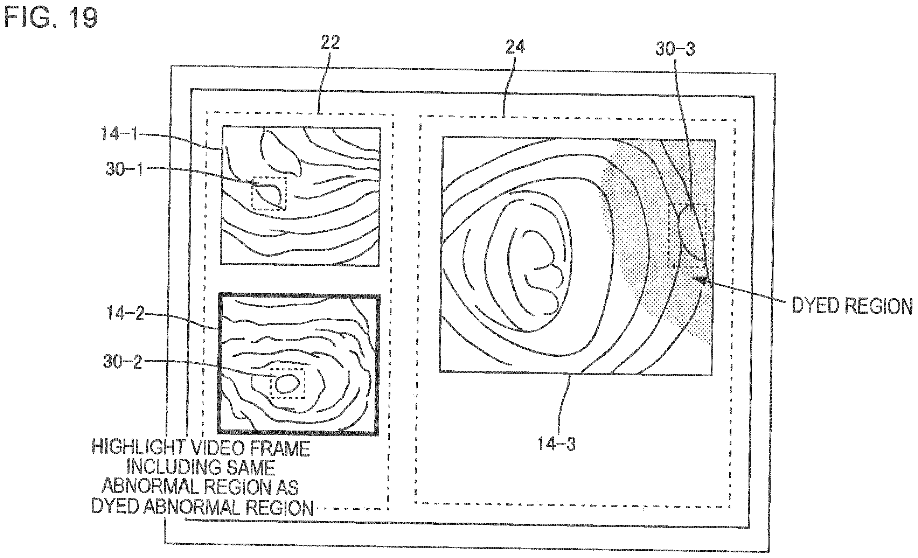

Fig. 19 is a diagram illustrating a scene in which the video frame 14 including the abnormal region 30 targeted for the predetermined action by the user is highlighted. The predetermined action by the user in this example is an action of dyeing the periphery of the abnormal region 30.

In Fig. 19, the abnormal region 30-3 is detected from the video frame 14-3 displayed in the second region 24. Further, the periphery of the abnormal region 30-3 is dyed.

Here, the abnormal region 30-2 representing the same abnormal region as the abnormal region 30-3 is detected from the video frame 14-2 among the video frames 14 displayed in the first region 22. The highlighting that thickens the frame line of the video frame 14-2 is performed.

Note that the processing performed when the predetermined action by the user is detected is not limited to the display of the third display described above. For example, the display control unit 2040 may not display the video frame 14 including the abnormal region 30 targeted for the predetermined action by the user in the first region 22. By doing this, in the first region 22, only the abnormal region 30 with a high probability of not being recognized by the user is displayed on the display device 20 among the video frames 14 in which the abnormal regions 30 are detected by the detection unit 2020. Accordingly, it is possible for the user to easily recognize the abnormal region 30 that was not recognized by the user.

### <Method of Detecting Predetermined Action by User>

The various predetermined actions described above are performed by the user performing a predetermined input operation on the endoscope system or the like. For example, in a common endoscope system, a scope provided with a camera is provided with a mechanism for irradiating light (such as a light source), a mechanism for spraying dye or coloring solution, a mechanism for administering water or a medicine, a mechanism for collecting a tissue, and the like. These mechanisms operate in response to a predetermined input operation by the user performed on the endoscope system. In other words, when the various predetermined actions described above are performed, the input operations for operating the mechanisms that realize the predetermined actions are performed.

For example, the second detection unit 2100 detects that the input operations for operating these mechanisms are performed in order to detect that the predetermined actions are performed by the user. For example, the second detection unit 2100 receives a notification indicating that the input operation is performed from the endoscope system or the like in order to detect that the input operation is performed.

Here, the second detection unit 2100 handles a site (site captured by the camera 10) included in the video frame 14 displayed in the second region 24 at a timing when the input operation is detected, as a site targeted for the predetermined action by the user. That is, in a case where the abnormal region 30 is included in the video frame 14 displayed in the second region 24 at the timing when the input operation is detected, the second detection unit 2100 handles the abnormal region 30 as the abnormal region 30 targeted for the predetermined action by the user.

Note that the method of the second detection unit 2100 detecting the predetermined action by the user is not limited to the above method of detecting the input operation. For example, the second detection unit 2100 may perform the image analysis of the video data 12 in order to detect the predetermined action by the user. For example, the second detection unit 2100 compares the brightness distribution or color distribution of each video frame 14 included in the video data 12 in order to detect a change in brightness or color of the imaging range of the camera 10. By doing this, the second detection unit 2100 detects that the color or intensity of the light illuminating the imaging range of the camera 10 is changed or the dyeing solution is sprayed.

In a case where the image analysis is used in this manner, for example, the second detection unit 2100 handles the site included in the video frame 14 that is detected to be changed in brightness or color, as the site targeted for the predetermined action by the user. That is, when the abnormal region 30 is included in the video frame 14 that is detected to be changed in brightness or color, the second detection unit 2100 handles this abnormal region 30 as the abnormal region 30 targeted for the predetermined action by the user.

### <Example of Hardware Configuration>

A hardware configuration of a computer that forms the information processing apparatus 2000 according to the example embodiment 4 is represented, for example, by Fig. 3 similar to the example embodiment 1. However, the storage device 1080 of the computer 1000 that forms the information processing apparatus 2000 according to the present example embodiment further stores a program module that realizes the functions of the information processing apparatus 2000 according to the present example embodiment.

As described above, the example embodiments according to the present invention are described with reference to the drawings, but these are the examples of the present invention. The present invention may employ a combination of the example embodiments described above or various configurations other than the above.

Some or all of the above example embodiments may be described as in the following additions, but are not limited to the additions.
1. An information processing apparatus including: a detection unit that detects an abnormal region in a body from a video in which the body is imaged; and a display control unit that displays a video frame in which the abnormal region is detected among video frames constituting the video in a first region of a display device and displays the video including the video frame generated after the video frame in a second region of the display device.
2. The information processing apparatus according to 1, in which the display control unit displays a first display indicating a position of the abnormal region in the video frame in which the abnormal region to be displayed on the display device is detected.
3. The information processing apparatus according to 2, in which the display control unit displays a plurality of the video frames in which the abnormal region is detected in the first region.
4. The information processing apparatus according to 3, further including: a deciding unit that decides whether abnormal regions detected from a plurality of video frames represent the same abnormality. The display control unit displays the same first display for the abnormal regions in a case where the abnormal regions detected from the plurality of video frames are decided to be the same, and displays different first displays for the abnormal regions in a case where the abnormal regions detected from the plurality of video frames are decided to be different from each other.
5. The information processing apparatus according to any one of 1 to 4, further including: a deciding unit that decides whether abnormal regions detected from a plurality of video frames are the same, in which the detection unit displays some video frames of the plurality of video frames in the first region in a case where the abnormal regions detected from the plurality of video frames are decided to be the same.
6. The information processing apparatus according to 5, in which the detection unit displays the video frame having the highest likelihood with which the abnormal region represents an abnormality, the video frame having the shortest distance between the abnormal region and the center position of the video frame, the video frame having the highest contrast in the entire image region, or the video frame having the highest contrast in the abnormal region in the first region, among the plurality of video frames in which the same abnonnal region is detected.
7. The information processing apparatus according to any one of 1 to 6, in which the detection unit stores the video frame in which the abnormal region is detected, in a storage unit.
8. The information processing apparatus according to 7, further including: a specification reception unit that receives an input specifying one of a plurality of video frames constituting the video and stores the specified video frame in the storage unit. The detection unit stores the video frame in which the abnormal region is detected in the storage unit so as to be discriminable from the video frame stored in the storage unit by the specification reception unit.
9. The information processing apparatus according to 8, further including: a deciding unit that decides whether abnormal regions detected from a plurality of video frames are the same. The display control unit displays a predetermined display in a first video frame to be displayed in the first region or a periphery of the first video frame in a case where a second video frame decided to include the same abnormal region as the abnormal region detected from the first video frame is specified as the input to the specification reception unit.
10. The information processing apparatus according to 8, further including: a deciding unit that decides whether the abnormal regions detected from a plurality of video frames are the same. The display control unit does not display a first video frame in the first region in a case where a second video frame decided to include the same abnormal region as the abnormal region detected from the first video frame is specified as the input to the specification reception unit.
11. The information processing apparatus according to any one of 1 to 10, further including: a second detection unit that detects a predetermined action by a user with respect to the detected abnormal region or a periphery of the abnormal region. The display control unit displays a predetermined display in the video frame including the detected abnormal region and to be displayed in the first region or a periphery of the video frame in a case where the predetermined action by the user with respect to the detected abnormal region or the periphery of the abnormal region is detected.
12. The information processing apparatus according to any one of 1 to 10, further including: a second detection unit that detects a predetermined action by a user with respect to the detected abnormal region or a periphery of the abnormal region. The display control unit does not display the video frame including the detected abnormal region in the first region in a case where the predetermined action by the user with respect to the detected abnormal region or the periphery of the abnormal region is detected.
13. The information processing apparatus according to 11 or 12, in which the predetermined action by the user is an action of changing a color or intensity of light irradiated to the detected abnormal region or the periphery of the abnormal region, an action of performing coloring agent spraying or dyeing in the detected abnormal region or the periphery of the abnormal region, an action of administering water or a medicine to the detected abnormal region or the periphery of the abnormal region, or an action of collecting a tissue of the detected abnormal region or the periphery of the abnormal region.
14. A control method executed by a computer, the method including: a detection step of detecting an abnormal region in a body from a video in which the body is imaged; and a display control step of displaying a video frame in which the abnormal region is detected among video frames constituting the video in a first region of a display device and displaying the video including the video frame generated after the video frame in a second region of the display device.
15. The control method according to 14, in which in the display control step, a first display indicating a position of the abnormal region is displayed in the video frame in which the abnormal region displayed on the display device is detected.
16. The control method according to 15, in which in the display control step, a plurality of the video frames in which the abnormal region is detected is displayed in the first region.
17. The control method according to 16, further including: a deciding step of deciding whether abnormal regions detected from a plurality of video frames represent the same abnormality. In the display control step, the same first display is displayed for the abnormal regions in a case where the abnormal regions detected from the plurality of video frames are decided to be the same, and different first displays are displayed for the abnormal regions in a case where the abnormal regions detected from the plurality of video frames are decided to be different from each other.
18. The control method according to any one of 14 to 17, further including: a deciding step of deciding whether abnormal regions detected from a plurality of video frames are the same. In the detection step, some video frames of the plurality of video frames are displayed in the first region in a case where the abnormal regions detected from the plurality of video frames are decided to be the same.
19. The control method according to 18, in which in the detection step, the video frame having the highest likelihood with which the abnormal region represents an abnormality, the video frame having the shortest distance between the abnormal region and the center position of the video frame, the video frame having the highest contrast in the entire image region, or the video frame having the highest contrast in the abnormal region are displayed in the first region, among the plurality of video frames in which the same abnormal region is detected.
20. The control method according to any one of 14 to 19, in which in the detection step, the video frame in which the abnormal region is detected is stored in a storage unit.
21. The control method according to 20, further including: a specification reception step of receiving an input specifying one of a plurality of video frames constituting the video and storing the specified video frame in the storage unit. In the detection step, the video frame in which the abnormal region is detected is stored in the storage unit so as to be discriminable from the video frame stored in the storage unit by the specification reception step.
22. The control method according to 21, further including: a deciding step of deciding whether abnormal regions detected from a plurality of video frames are the same. In the display control step, a predetermined display is displayed in a first video frame to be displayed in the first region or a periphery of the first video frame in a case where a second video frame decided to include the same abnormal region as the abnormal region detected from the first video frame is specified as the input to the specification reception step.
23. The control method according to 21, further including: a deciding step of deciding whether the abnormal regions detected from a plurality of video frames are the same. In the display control step, a first video frame is not displayed in the first region in a case where a second video frame decided to include the same abnormal region as the abnormal region detected from the first video frame is specified as the input to the specification reception step.
24. The control method according to any one of 14 to 23, further including: a second detection step of detecting a predetermined action by a user with respect to the detected abnormal region or a periphery of the abnormal region. In the display control step, a predetermined display is displayed in the video frame including the detected abnormal region and to be displayed in the first region or a periphery of the video frame in a case where the predetermined action by the user with respect to the detected abnormal region or the periphery of the abnormal region is detected.
25. The control method according to any one of 14 to 23, further including: a second detection step of detecting a predetermined action by a user with respect to the detected abnormal region or a periphery of the abnormal region. In the display control step, the video frame including the detected abnormal region is not displayed in the first region in a case where the predetermined action by the user with respect to the detected abnormal region or the periphery of the abnormal region is detected.
26. The control method according to 24 or 25, in which the predetermined action by the user is an action of changing a color or intensity of light irradiated to the detected abnormal region or the periphery of the abnormal region, an action of performing coloring agent spraying or dyeing in the detected abnormal region or the periphery of the abnormal region, an action of administering water or a medicine to the detected abnormal region or the periphery of the abnormal region, or an action of collecting a tissue of the detected abnormal region or the periphery of the abnormal region.
27. A program causing a computer to execute each step of the control method according to any one of 14 to 26.

This application claims priority based on Japanese Patent Application No. 2017-103348 filed on May 25, 2017, the entire disclosure of which is incorporated herein.

## Claims

1. An information processing apparatus comprising:
a detection unit that detects an abnormal region in an inside of a body from a video in which the inside of the body is imaged; and
a display control unit that displays a video frame from which the abnormal region is detected among video frames constituting the video in a first region of a display device, and displays the video including the video frame generated after the video frame in a second region of the display device.

2. The information processing apparatus according to claim 1,
wherein the display control unit displays a first display indicating a position of the abnormal region in the video frame from which the abnormal region to be displayed on the display device is detected.

3. The information processing apparatus according to claim 2,
wherein the display control unit displays a plurality of the video frames from which the abnormal region is detected in the first region.

4. The information processing apparatus according to claim 3, further comprising:
a deciding unit that decides whether abnormal regions detected from a plurality of video frames represent a same abnormality,
wherein the display control unit performs:
displaying a same first display for the abnormal regions when the abnormal regions detected from the plurality of video frames are decided to be same, and
displays different first displays for the abnormal regions when the abnormal regions detected from the plurality of video frames are decided to be different from each other.

5. The information processing apparatus according to any one of claims 1 to 4, further comprising:
a deciding unit that decides whether abnormal regions detected from a plurality of video frames are same,
wherein the detection unit displays some video frames of the plurality of video frames in the first region when the abnormal regions detected from the plurality of video frames are decided to be same.

6. The information processing apparatus according to claim 5,
wherein the detection unit displays the video frame having the highest likelihood of that the abnormal region represents an abnormality, the video frame having the shortest distance between the abnormal region and the center position of the video frame, the video frame having the highest contrast in the entire image region, or the video frame having the highest contrast in the abnormal region in the first region, among the plurality of video frames from which a same abnormal region is detected.

7. The information processing apparatus according to any one of claims 1 to 6,
wherein the detection unit records the video frame from which the abnormal region is detected in a storage unit.

8. The information processing apparatus according to claim 7, further comprising:
a specification reception unit that receives an input specifying one of a plurality of video frames constituting the video and records the specified video frame in the storage unit,
wherein the detection unit records the video frame from which the abnormal region is detected in the storage unit so as to be discriminable from the video frame recorded in the storage unit by the specification reception unit.

9. The information processing apparatus according to claim 8, further comprising:
a deciding unit that decides whether abnormal regions detected from a plurality of video frames are same,
wherein, when a second video frame is specified as the input to the specification reception unit, the display control unit displays a predetermined display in a first video frame displayed in the first region or in a periphery of the first video frame, the second video frame being decided to include a same abnormal region as the abnormal region detected from the first video frame.

10. The information processing apparatus according to claim 8, further comprising:
a deciding unit that decides whether the abnormal regions detected from a plurality of video frames are same,
wherein the display control unit does not display a first video frame in the first region when a second video frame is specified as the input to the specification reception unit, the second video frame being decided to include the same abnormal region as the abnormal region detected from the first video frame.

11. The information processing apparatus according to any one of claims 1 to 10, further comprising:
a second detection unit that detects a predetermined action by a user to the detected abnormal region or a periphery of the abnormal region,
wherein, when the predetermined action to the detected abnormal region or the periphery of the abnormal region is detected, the display control unit displays a predetermined display in the video frame that includes the detected abnormal region and is displayed in the first region, or in a periphery of the video frame.

12. The information processing apparatus according to any one of claims 1 to 10, further comprising:
a second detection unit that detects a predetermined action by a user to the detected abnormal region or a periphery of the abnormal region,
wherein the display control unit does not display the video frame including the detected abnormal region in the first region when the predetermined action by the user to the detected abnormal region or the periphery of the abnormal region is detected.

13. The information processing apparatus according to claim 11 or 12,
wherein the predetermined action by the user is an action of changing a color or intensity of light irradiated to the detected abnormal region or the periphery of the abnormal region, an action of performing dye spraying or coloring in the detected abnormal region or the periphery of the abnormal region, an action of administering water or a medicine to the detected abnormal region or the periphery of the abnormal region, or an action of collecting a tissue of the detected abnormal region or the periphery of the abnormal region.

14. A control method executed by a computer, the method comprising:
a detection step of detecting an abnormal region in an inside of a body from a video in which the inside of the body is imaged; and
a display control step of displaying a video frame from which the abnormal region is detected among video frames constituting the video in a first region of a display device, and displaying the video including the video frame generated after the video frame in a second region of the display device.

15. The control method according to claim 14, further comprising:
in the display control step, displaying a first display indicating a position of the abnormal region in the video frame from which the abnormal region to be displayed on the display device is detected.

16. The control method according to claim 15, further comprising:
in the display control step, displaying a plurality of the video frames from which the abnormal region is detected in the first region.

17. The control method according to claim 16, further comprising:
a deciding step of deciding whether abnormal regions detected from a plurality of video frames represent a same abnormality,
in the display control step, performing:
displaying a same first display for the abnormal regions when the abnormal regions detected from the plurality of video frames are decided to be the same, and
displaying different first displays for the abnormal regions when the abnormal regions detected from the plurality of video frames are decided to be different from each other.

18. The control method according to any one of claims 14 to 17, further comprising:
a deciding step of deciding whether abnormal regions detected from a plurality of video frames are same,
in the detection step, displaying some video frames of the plurality of video frames in the first region when the abnormal regions detected from the plurality of video frames are decided to be same.

19. The control method according to claim 18, further comprising:
in the detection step, displaying the video frame having the highest likelihood of that the abnormal region represents an abnormality, the video frame having the shortest distance between the abnormal region and the center position of the video frame, the video frame having the highest contrast in the entire image region, or the video frame having the highest contrast in the abnormal region are displayed in the first region, among the plurality of video frames from which a same abnormal region is detected.

20. The control method according to any one of claims 14 to 19, further comprising:
in the detection step, recording the video frame from which the abnormal region is detected in a storage unit.

21. The control method according to claim 20, further comprising:
a specification reception step of receiving an input specifying one of a plurality of video frames constituting the video and recording the specified video frame in the storage unit,
in the detection step, recoding the video frame from which the abnormal region is detected in the storage unit so as to be discriminable from the video frame recorded in the storage unit by the specification reception step.

22. The control method according to claim 21, further comprising:
a deciding step of deciding whether abnormal regions detected from a plurality of video frames are same,
in the display control step, when a second video frame is specified as the input to the specification reception unit, displaying a predetermined display in a first video frame displayed in the first region or in a periphery of the first video frame, the second video frame being decided to include a same abnormal region as the abnormal region detected from the first video frame.

23. The control method according to claim 21, further comprising:
a deciding step of deciding whether the abnormal regions detected from a plurality of video frames are same,
wherein in the display control step, a first video frame is not displayed in the first region when a second video frame is specified as the input in the specification reception step, the second video frame being decided to include the same abnormal region as the abnormal region detected from the first video frame.

24. The control method according to any one of claims 14 to 23, further comprising:
a second detection step of detecting a predetermined action by a user to the detected abnormal region or a periphery of the abnormal region,
in the display control step, when the predetermined action to the detected abnormal region or the periphery of the abnormal region is detected, displaying a predetermined display in the video frame that includes the detected abnormal region and is displayed in the first region, or in a periphery of the video frame.

25. The control method according to any one of claims 14 to 23, further comprising:
a second detection step of detecting a predetermined action by a user to the detected abnormal region or a periphery of the abnormal region,
wherein in the display control step, the video frame including the detected abnormal region is not displayed in the first region when the predetermined action by the user to the detected abnormal region or the periphery of the abnormal region is detected.

26. The control method according to claim 24 or 25,
wherein the predetermined action by the user is an action of changing a color or intensity of light irradiated to the detected abnormal region or the periphery of the abnormal region, an action of performing dye spraying or coloring in the detected abnormal region or the periphery of the abnormal region, an action of administering water or a medicine to the detected abnormal region or the periphery of the abnormal region, or an action of collecting a tissue of the detected abnormal region or the periphery of the abnormal region.

27. A program causing a computer to execute each step of the control method according to any one of claims 14 to 26.
